# EUROPÄISCHE PATENTANMELDUNG

(11) **EP 2 377 574 A2**
(43) Veröffentlichungstag der Anmeldung: **19.10.2011**
(21) Anmeldenummer: 11159331.5
(22) Anmeldetag: 23.03.2011
(51) Int. Cl.: A61N 1/40, A61F 7/12, A61K 41/00, A61B 5/07, H02J 17/00, A61K 9/00

(54) **Implantat und Applikator**

(30) Priorität: 13.04.2010 US 323361 P
(71) Anmelder: BIOTRONIK SE & Co. KG, 12359 Berlin (DE)
(72) Erfinder: Dörr, Thomas, 12437, Berlin (DE)
(74) Vertreter: Lindner-Vogt, Karin L.

(57) **Zusammenfassung**

Die Erfindung betrifft ein Implantat mit einem Energiewandler, bei dem der Energiewandler ausgebildet ist eingeprägte Energie in Wärme zu wandeln, wobei der Energiewandler angeordnet ist, dass infolge der Energiewandlung entstehende Wärme an einer Außenfläche des Implantats wirksam wird.

## Beschreibung

Die Erfindung betrifft ein Implantat mit einem Energiewandler, und zwar speziell ein Mikroimplantat für beispielsweise eine lokale Medikamentenabgabe oder ähnliches.

Bei derartigen Implantaten besteht grundsätzlich das Problem, diese an einem bestimmten Ort in einem Körper zu fixieren.

Es sind viele verschiedene "mechanische" Fixationsmethoden für aktive und passive Implantate bekannt.

Keine der vorbekannten Lösungen bezieht sich jedoch auf die Fixierung von relativ kleinen Implantaten in einem Zielorgan, welches entweder keine festen (z.B. muskulären) Strukturen aufweist oder Blutgefäße zur Fixierung mittels Stent o. ä. aufweisen. Eine Fixierung beispielsweise eines Medikamenten-eluierenden Implantats innerhalb eines Lebertumors oder eines RF-Sensorimplantates im Lungengewebe ist derzeit nicht bekannt.

Bislang waren derartige Implantate nicht bekannt, so dass auch die Fixierung in o. g. Gewebestrukturen nicht notwendig war. Allerdings werden derartige Anwendungen mit dem Fortschreiten der RF-Technologie und anderen Technologie, wie die Verpackung von Medikamenten in magnetischen Nanostrukturen immer wichtiger und das Problem der Fixierung muss zukünftig gelöst werden.

Ziel der vorliegenden Erfindung ist es beispielsweise zu ermöglichen, dass mikro- und nanometrisch verpackte Medikamentendepots oder Medikamente zuverlässig, wiederholt und einfach an einem entsprechend definierten Wirkort konzentriert werden können. Im Vordergrund steht dabei eine gut reproduzierbare Anwendung

Erfindungsgemäß wird dieses Ziel durch ein Implantat mit einem Energiewandler erreicht, bei dem der Energiewandler ausgebildet ist, vorzugsweise berührungsfrei eingeprägte Energie in Wärme zu wandeln, und wobei der Energiewandler in Bezug auf das übrige Implantat derart angeordnet ist, dass infolge der Energiewandlung entstehende Wärme an einer Außenfläche des Implantat wirksam wird.

Ein solches Implantat erlaubt es, von außen berührungslos Energie einzuprägen, die dann durch den Energiewandler in Wärme umgesetzt wird. Die Wärme wird an wenigstens einer Außenfläche des Implantats wirksam und führt zu einer Gewebekoagulation. Die Gewebekoagulation bewirkt eine Blutgerinnung infolge einer Nekrose des Gewebes, die wiederum zu einer Einkapselung des Implantats und damit zu einer Fixierung im Zielorgan führt.

Entsprechend ist der Energiewandler so ausgebildet, dass er eine ausreichend hohe Leistung aufnehmen und entsprechend eine ausreichend hohe Wärmeleistung abgeben kann, um eine derartige Gewebekoagulation zu bewirken.

Zur Unterstützung einer derartigen Einkapselung des Implantats kann das Implantat gemäß einer bevorzugten Ausführungsvariante mit einem gerinnungsfördernden Wirkstoff beschichtet sein, um so die Blutungskomplikation im Zielorgan, beispielsweise stark durchbluteten Tumoren, zu verringern. Dementsprechend kann die Beschichtung als Wirkstoff Gerinnungsfaktoren und insbesondere Vitamin K und/oder Aprotinin enthalten.

Es entsteht so ein dauerhaft im menschlichen Körper und seinen Organen implantierbares aktives oder passives Implantat, das eine Oberfläche oder eine Oberflächenbeschichtung besitzt, die eine Einkapselung des Implantats in das umliegende Gewebe bewirkt.

Das Implantat kann beispielsweise ein Permanentmagnet sein oder aus einem magnetisierbaren und entmagnetisierbaren Material bestehen, um auf diese Weise mikro-oder namometrisch verpackte Medikamentendepots an einem gewünschten Wirkort zu konzentrieren. Diese Medikamentendepots bestehen bzw. beinhalten ferromagnetische Trägerstrukturen, die eine Medikament aufnehmen können und über den Blutkreislauf im Körper zirkulieren. Die Freisetzung der Wirkstoffe erfolgt entweder durch eine definierte Elution oder durch Auflösung der biodegradierbaren Trägerstruktur (z.B. Fe₃O₄) Ist das Implantat aus einem entmagnetisierbaren Material, so kann im Rahmen einer sequenziellen Therapie wahlweise ein Wirkort für die Therapie "hinzu- oder abgeschaltet" werden.

Außerdem besteht das Implantat vorzugsweise aus einem biodegradierbaren Material, so dass das Implantat im Laufe der Zeit vom Körper abgebaut wird.

Auch können an dem Implantat Medikamentendepots vorgesehen sein, die Medikamente enthalten können, die im Laufe der Zeit nach der Implantation freigesetzt, d.h. eluiert werden.

Das Implantat kann auch als aktives Implantat mit einer Medikamentenpumpe oder einem gezielt freizugebenden Medikamentendepot sein.

Zusätzlich oder alternativ kann das Implantat auch ein aktives oder ein passives Sensorimplantat zum Überwachen eines oder mehrerer physiologischer Parameter sein. In diesem Falle könnte das Implantat beispielsweise ein an sich bekanntes RFID-Implantat sein.

Zum Erreichen des eingangs genannten Ziels wird auch eine Kombination aus einem Implantat der hierin zuvor beschriebenen Art mit einem Applikator vorgeschlagen, der zum einen zum Einführen des Implantats in den Körper dient und darüber hinaus vorzugsweise auch eine Einkopplung oder Einprägung von Energie in das Implantat ermöglicht. Ein derartiger Applikator wird nur temporär zum Einführen und Fixieren des Implantates genutzt und kann nach der Implantation entfernt werden.

Zusätzlich oder alternativ kann der Applikator auch ein Biopsiegerät sein, mit dem gleichzeitig eine Gewebeprobe entnommen und das Implantat appliziert werden kann. Gemäß einer bevorzugten Ausführungsvariante ist der Applikator außerdem dazu ausgebildet, über den Applikator Energie in das Implantat einzuprägen, um so eine Gewebefixierung zu bewirken.

Gemäß einer Variante ist der Energiewandler dazu ausgebildet, über ein elektromagnetisches Wechselfeld eingeprägte elektrische Leistung aufzunehmen. Der Energiewandler enthält in diesem Falle ein elektrisches Heizelement, das die aufgenommene elektrische Leistung in Wärmeleistung wandelt. Zum Aufnehmen von Leistung aus einem elektromagnetischen Wechselfeld besitzt der Wandler vorzugsweise eine Antenne oder ist mit einer Antenne zu verbinden. Die Antenne kann Teil eines Resonators sein, der auf eine Frequenz eines elektromagnetischen Wechselfeldes abgestimmt ist.

Alternativ hierzu kann der Energiewandler auch eine Spule aufweisen, so dass elektrische Leistung induktiv, d.h. über ein im Wesentlichen magnetisches Feld, einzuprägen ist.

Gemäß einer weiteren Alternative kann der Energiewandler auch zum Wandeln von Schallenergie in Wärmeenergie ausgebildet sein und dazu einen entsprechenden mechanischen Resonator aufweisen, der auf die Frequenz eingeprägter Ultraschallenergie abgestimmt ist.

Im Falle eines Implantates, dessen Energiewandler zur Aufnahme von Energie über ein hochfrequentes elektromagnetisches Wechselfeld ausgebildet ist, besitzt der Energiewandler vorzugsweise eine Antenne oder ist mit einer solchen zu verbinden (siehe oben). Die Antenne kann dabei beispielsweise Teil eines Applikators sein (siehe ebenfalls oben) oder ein separates Teil, das beispielsweise aus resorbierbaren Materialien besteht. In diesem Falle könnte die Antenne einen Isolator aus einem degradierbaren Polymer und einen Leiter aus einem degradierbaren Metall, wie beispielsweise Magnesium, enthalten.

Das Implantat ist vorzugsweise kürzer als 10 cm und damit ausreichend klein, um als MRT-kompatibel zu gelten. Die mit dem Implantat und dessen Energiewandler verbundene Antenne kann dabei länger sein, insbesondere wenn die Antenne - wie zuletzt gesagt - bioresorbierbar ausgeführt ist, so dass das Implantat nach Degradation der Antenne wieder MRT-kompatibel ist. Wie zuvor erwähnt, ist das Implantat vorzugsweise ein sehr kleines Implantat mit einer Masse von weniger als 10 g und besonders bevorzugt weniger als 1 g. Das Volumen des Implantates ist vorzugsweise kleiner als 10 cm³ und besonders bevorzugt kleiner als 1 cm³. Noch kleinere Implantate mit einem Volumen von weniger als 10 mm³ oder besser noch weniger als 1 mm³ sind ganz besonders bevorzugt und eignen sich insbesondere als Medikamententräger für die lokale Medikamentenapplikation.

Gemäß einer bevorzugten Ausführungsaltemative ist das Implantat mit einer Extraktionshilfe verbunden, die es erlaubt, das Implantat beispielsweise nach erfolgter Medikamentenabgabe oder abgeschlossenem Monitoring wieder zu entfernen. Eine derartige Extraktionshilfe ist dann nicht erforderlich, wenn - wie zuvor dargestellt - das Implantat vollständig aus bioresorbierbarem oder biodegradierbarem Material besteht.

Vorzugsweise sind das Implantat und sein Energiewandler dazu ausgebildet, eine Hyperthermie-Therapie auszuführen, also eine Therapie, die auf einer Erwärmung des Gewebes über die natürliche Körpertemperatur hinaus beruht, ohne dass es dabei zu einer Koagulation kommt. In diesem Falle können das Implantat und der Energiewandler in vorteilhafter Weise sowohl zur Fixierung des Implantats mittels Gewebekoagulation durch entsprechende Überhitzung des Gewebes dienen, um dann anschließend nach erfolgter Fixierung eine Hyperthermie-Therapie mit dem Implantat durchzuführen. Für den Zweck der Gewebenekrotisierung per Koagulation und die Hyperthermie-Therapie können aber auch unterschiedliche Energiewandler vorgesehen sein, um die jeweiligen Energiewandler besser auf den jeweiligen speziellen Zweck abstimmen zu können.

Im Folgenden soll nun die Erfindung in Bezug auf die Figuren näher erläutert werden. Von den Figuren zeigt:
- Figur 1:: ein erfindungsgemäßes Implantat in schematischer Darstellung;
- Figur 2:: mehrere Implantate in einem Zielorgan;
- Figur 3:: einen medikamentenbeschichtete, implantierbaren Magnet;
- Figur 4:: einen Applikator mit Implantat für die die HF-Koagulation; und
- Figur 5:: ein Implantat mit HF-Antenne

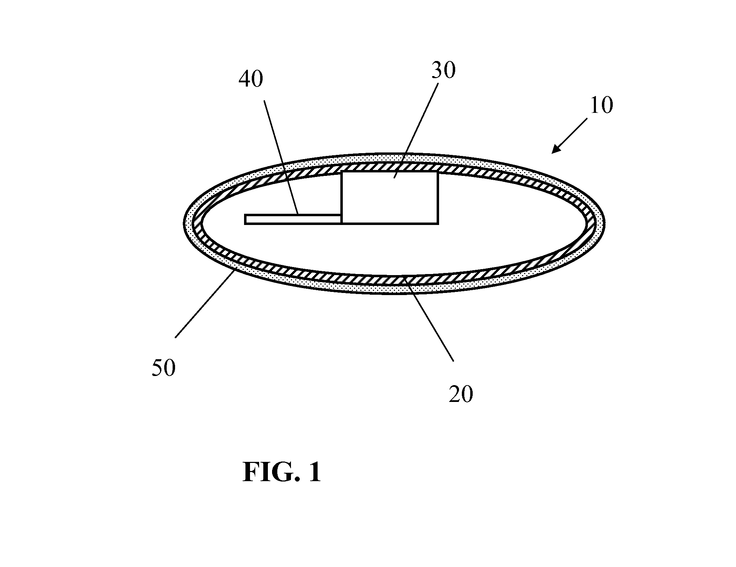

Fig. 1 zeigt ein erfindungsgemäßes Implantat 10, das als Mikroimplantat mit einem wärmeleitenden Gehäuse 20 ausgebildet ist und einen Energiewandler 30 aufweist, der seinerseits wieder einen Energieempfänger 40 aufweist oder mit diesem verbunden ist. Der Energieempfänger 40 kann ein mechanischer Resonator sein oder eine Antenne zum Empfang hochfrequenter elektromagnetischer Strahlung oder auch einer Spule zur Induktion von Leistung auf magnetischem Wege. Die über ein hochfrequentes elektromagnetisches Wechselfeld über eine Antenne empfangene Leistung oder die über eine Spule auf dem Wege der Induktion empfangene Leistung steht im Ergebnis als elektrische Leistung zur Verfügung. In diesem Falle enthält der Energiewandler 30 vorzugsweise ein elektrisches Heizelement, das die elektrische Leistung in Wärme wandelt. Ein derartiges elektrisches Heizelement könnte beispielsweise nach Art eines Ohm'schen Widerstandes aufgebaut sein.

Der Energieempfänger 40 ist mit dem eigentlichen Energiewandler 30 derart verbunden, dass über den Energieempfänger 40 empfangene Energie von dem Energiewandler 30 in Wärme zu wandeln ist. Dabei ist der Energiewandler 30 so mit dem Gehäuse 20 des Implantats 10 verbunden, dass die infolge der Energiewandlung entstehende Wärme über das Gehäuse 20 des Implantats 10 an umliegendes Gewebe abgegeben wird.

Der Energiewandler 30 und der Energieempfänger 40 sowie auch das Implantat 10 selbst sind so ausgebildet, dass das Implantat 10 über den Energieempfänger 40 genügend Leistung aufnehmen kann und der Energiewandler diese Leistung in Wärme wandelt, die ausreicht, um in der unmittelbaren Umgebung des Implantats 10 eine Gewebekoagulation und damit im Ergebnis eine Gewebenekrose zu bewirken, die zu einer Einkapselung des Implantats 10 und damit zu einer Fixierung des Implantats 10 führt.

Das in Fig. 1 abgebildete Implantat 10 ist in der dargestellten bevorzugten Ausführungsvariante eine Medikamentenbeschichtung 50.

Bei dieser Medikamentenbeschichtung 50 kann es sich um einen therapeutischen Wirkstoff handeln, beispielsweise zur Tumorbekämpfung. Zusätzlich oder alternativ kann die Medikamentenbeschichtung 50 jedoch auch einen Gerinnungsfaktor und insbesondere Vitamin K und/oder Aprolinin enthalten, die die Blutgerinnung fördern und damit einerseits Blutungen im Tumorgewebe hemmen und so einer Verschleppung von Tumorzellen entgegenwirken und andererseits auch die Fixierung des Implantats fördern.

In diesem Zusammenhang ist darauf hinzuweisen, dass die Fixation des Implantats durch Einkapselung infolge Gewebekoagulation auch den Vorteil mit sich bringt, dass hierdurch ebenfalls eine Verschleppung von Tumorzellen vermindert wird.

Wie eingangs bereits erwähnt, kann das Implantat 10 darüber hinaus gezielte Medikamentendepots enthalten oder auch als aktives oder passives Sensorimplantat, beispielsweise als RFID-Implantat ausgebildet sein. Alle Bestandteile des Implantats 10 können aus biodegradierbarem oder bioresorbierbarem Material bestehen und beispielsweise biodegradierbare Metalle, wie Magnesium, enthalten. Mögliche Sensoren für ein solches Implantat sind Sensoren zur lokalen Druckmessung, potentiometrische pH-Wert-Sensorsen oder Temperatursensoren.

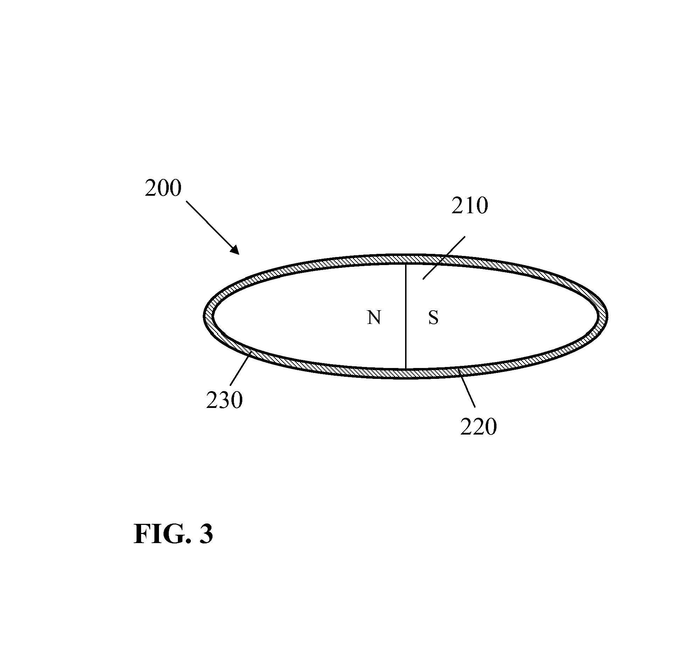

Wie nachfolgend in Bezug auf Fig. 3 noch näher erläutert, kann das Implantat 10 auch magnetisch sein, so dass ferromagnetische Medikamentendepots magnetisch an das Implantat 10 gekoppelt sein können bzw. dieses injizierte magnetische, nanometrisch verpackte Medikamentendepots anziehen kann.

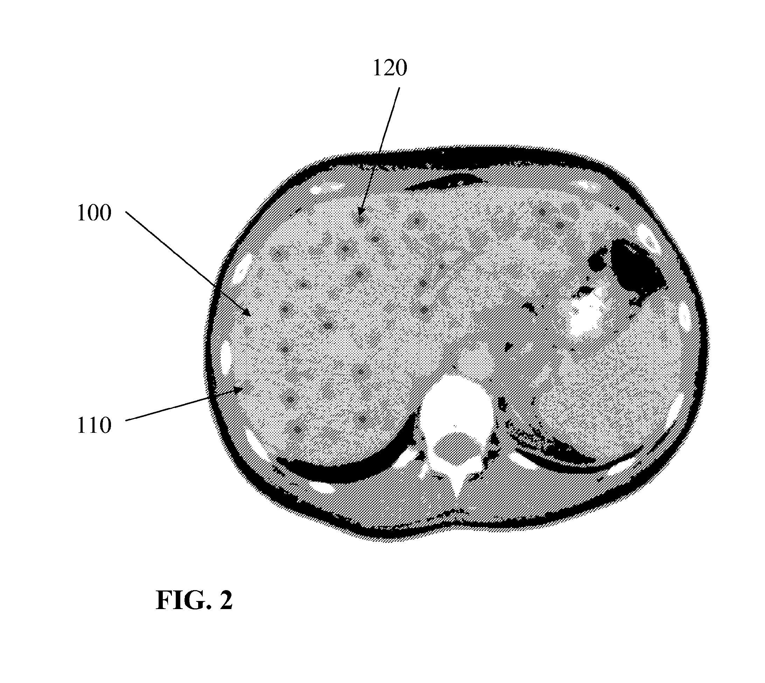

In der Figur 2 ist ein Magnet-Resonanz- Tomogram der Leber gezeigt. In diesem Fall ist die Leber 100 mit mehreren Metastasen 110 durchsetzt, die vom gesunden Gewebe noch gut abtrennbar sind. Zur Konzentration der Tumormedikation sind in die Metastasen mehrere Implantate 120 in Form von Permanentmagneten in Punktionstechnik implantiert, die eine tumorwirksame, in ferromagnetischen, resorbierbaren Nano-Medikamentendepots eingeschlossene Medikation im Zielgebiet konzentrieren. Nach Auflösung der resorbierbaren Depots (z.B. aus Fe₃O₄) erfolgt dann die stark lokale Wirkung der normalerweise stark systemisch wirksamen Substanzen.

In der Figur 3 ist ein Implantat in Form eines implantierbaren Magneten 200 dargestellt, dessen permanentmagnetischer Kern 210 mit einem gerinnungsfördernden Medikament (z.B. Gerinnungsfaktoren, Vitamin K oder Aprotin) 220 beschichtet ist. Diese Beschichtung wiederum ist von einer bioresorbierbaren Schutz- und Gleitschicht 230 umgeben, so dass der medikamentöse Wirkstoff erst nach Applikation in das Zielorgan abgegeben wird.

Die Dimensionen des dargestellten implantierbaren Magneten betragen z.B. hinsichtlich der Länge weniger als 3mm und hinsichtlich des Durchmessers weniger als 1mm. Das bevorzugte Material für den Magneten ist eine Neodym-Verbindung, beschichtet mit einem biokompatiblen Material.

Das Ziel der medikamentösen Beschichtung besteht darin dass das Blutungsrisiko bei Implantation in einem stark durchbluteten Tumorgewebe minimiert wird und eine nekrotische Kapsel um das Implantat gebildet wird, so dass damit eine Fixation des implantierbaren Magneten stattfindet. Der implantierbare Magnet selbst dient der Medikamentenkonzentration in einem therapeutischen Zielgebiet, indem die Medikamente, gebunden an nanometrische magnetische Trägersubstanzen injiziert werden.

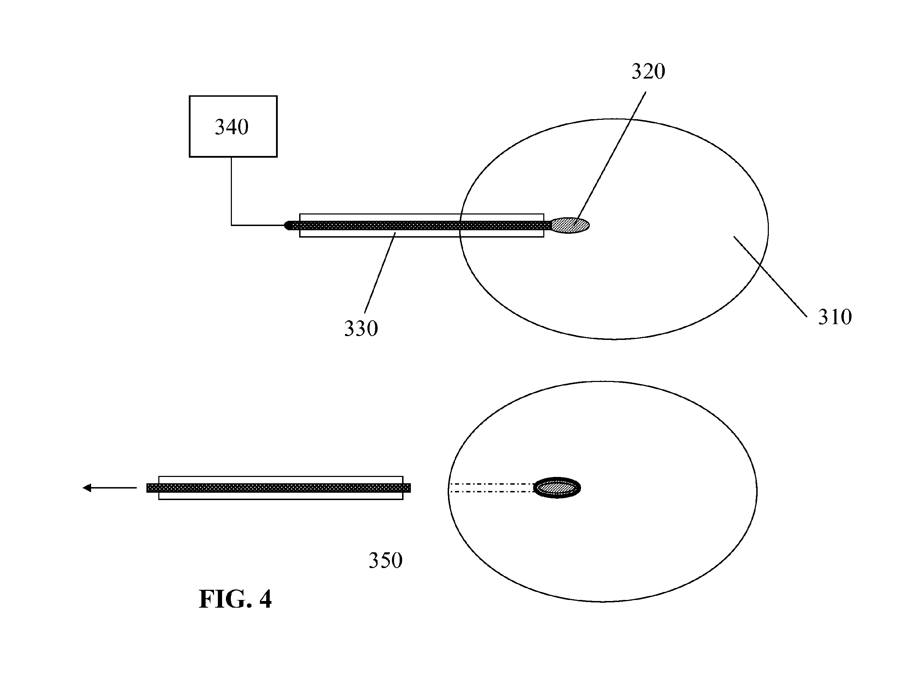

In der Figur 4 ist ein Zielorgan 310 dargestellt, in das ein Implantat 320 mittels eines Applikators 330 durch Punktionstechnik implantiert wurde. Zur Fixation des Implantates wird dem Implantat mittels eines angeschlossenen HF-Generators 340 Energie derart zugeführt, dass eine Erwärmung des Implantates und des umgebenden Gewebes stattfindet und so eine schnelle Nekrotisierung gefördert wird.

Die Verbindung zwischen Applikator und Implantat ist trennbar (z.B. Schraubverbindung o. ä.), so dass anschließend der Applikator entfernt werden kann (siehe 350).

Der Applikator ist dabei optional so gestaltet, dass dieser als Biopsiegerät fungiert. In dieser Ausführung besteht dieser aus einer äußeren Biopsienadel und austauschbaren Innenapplikatoren. Diese sind für die Biopsie in der gängigen Form eines Biopsiegerätes geformt und dienen der Gewebeentnahme. Nach der Biopsie kann an die identische Punktionsstelle das Implantat mittels eines Applikators positioniert werden, wobei der Applikator elektrisch mit dem Implantat verbunden bleibt, so dass die HF-Energie in das Implantat eingeprägt werden kann. Der innere Applikator ist elektrisch von der äußeren Biopsienadel getrennt, so dass die als Gegenelektrode für den HF-Generator dienen kann.

In einer alternativen Ausführung erfolgt die Energieeinprägung in das Implantat mittels fokusierter Ultraschallenergie (HIFU: High-intensity focussed ultrasound). In diesem Fall ist der Energiewandler zum Wandeln von Schallenergie in Wärmeenergie ausgebildet und weist vorzugsweise einen Resonator auf, der auf die Frequenz eingeprägter Ultraschallenergie abgestimmt ist.

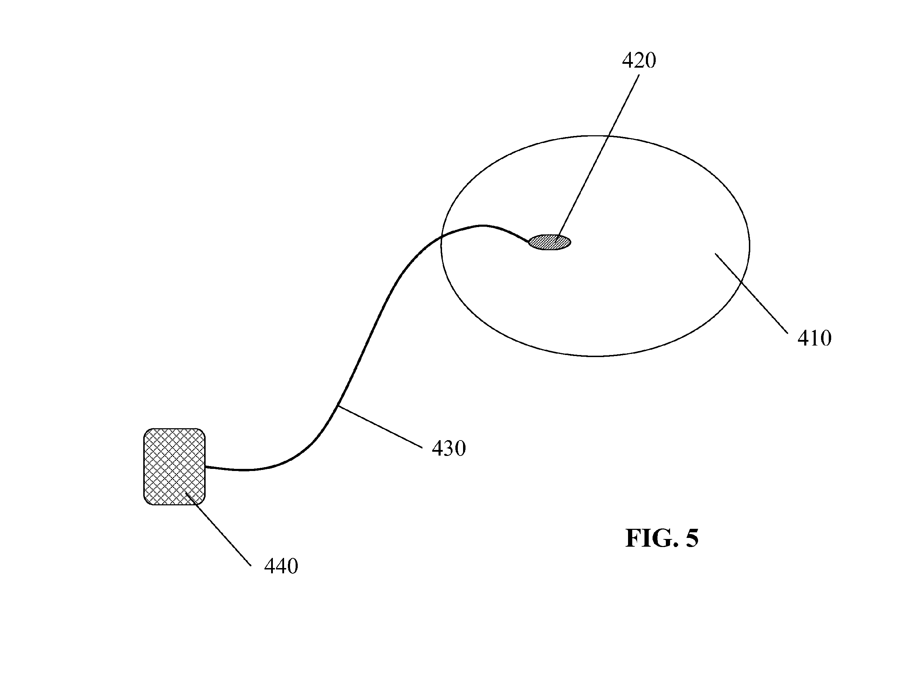

Bei dem in Figur 5 dargestellten Implantat 420 kann es sich z.B. handeln um:
- eine implantierbare Medikamentenpumpe mit eigener Energieversorgung oder
- eine implantierbare Medikamentenpumpe zur Bedarfsmedikation ohne eigene Energieversorgung, gesteuert durch externe Energieeinkopplung oder
- ein Implantat zur Hyperthermietherapie, dass eine gezielte Erwärmung von Gewebe mittels Umwandlung externer eingeprägter Energie bewirkt, wobei hier bevorzugt eine Energieumwandlung eines MRT-Systems genutzt werden kann und die Therapiesteuerung dabei durch unterschiedliche MRT-Frequenzen oder steuerbare Demodulatoren im Hyperthermieimplantat realisiert wird oder
- ein Sensorimplantat mit eigener Energieversorgung und Telemetriefunktion (z.B. Glucose oder pH-Sensor) oder
- ein Sensorimplantat ohne eigene Energieversorgung mit externer Abfragefunktion (z.B. mittels Ultraschall) oder
- ein implantierbares Medikamentendepot mit einer festen oder steuerbaren Elutionsrate.

Des Weiteren sind in der Figur 5 dargestellt:
das Zielorgan 410,
eine Antennenleitung zur Einprägung von HF-Energie, kann gleichzeitig als Extraktionshilfe dienen 430 und
eine flächige Gegenelektrode für die HF-Koagulation, Fläche ist deutlich größer als die Implantatsoberfläche, so dass eine relevante Erwärmung nur am Implantat erfolgt 440.

## Patentansprüche

1. Implantat mit einem Energiewandler, **dadurch gekennzeichnet, dass** der Energiewandler ausgebildet ist eingeprägte Energie in Wärme zu wandeln, wobei der Energiewandler angeordnet ist, dass infolge der Energiewandlung entstehende Wärme an einer Außenfläche des Implantats wirksam wird.

2. Implantat nach Anspruch 1, **dadurch gekennzeichnet, dass** der Energiewandler eine elektrisches Heizelement ist.

3. Implantat nach Anspruch 2, **dadurch gekennzeichnet, dass** der Energiewandler eine Antenne aufweist oder mit einer Antenne verbunden ist, die zum Empfang mittels eines elektromagnetischen Wechselfeldes eingeprägter elektrischer Leistung ausgebildet und mit dem elektrischen Heizelement derart verbunden ist, dass empfangene elektrische Leistung in dem elektrischen Heizelement in Wärme gewandelt wird.

4. Implantat nach Anspruch 1, **dadurch gekennzeichnet, dass** der Energiewandler zum Wandeln von Schallenergie in Wärmeenergie ausgebildet ist.

5. Implantat nach Anspruch 4, **dadurch gekennzeichnet, dass** der Energiewandler einen Resonator aufweist, der auf die Frequenz eingeprägter Ultraschallenergie abgestimmt ist.

6. Implantat nach einem der Ansprüche 1 bis 5, **dadurch gekennzeichnet, dass** das Implantat mit einem gerinnungsfördernden Wirkstoff beschichtet ist.

7. Implantat nach einem der Ansprüche 1 bis 6, **dadurch gekennzeichnet, dass** das Implantat ist ein Permanentmagnet ist.

8. Implantat nach einem der Ansprüche 1 bis 7, **dadurch gekennzeichnet, dass** das Implantat ein magnetisierbares und entmagnetisierbares Material zur magnetischen Medikamentenadhäsion enthält.

9. Implantat nach einem der Ansprüche 1 bis 6, **dadurch gekennzeichnet, dass** das Implantat aus biodegradierbaren Material besteht.

10. Implantat nach einem der Ansprüche 1 bis 9, **dadurch gekennzeichnet, dass** das Implantat Medikamentendepots zur Medikamentenfreisetzung besitzt.

11. Implantat nach einem der Ansprüche 1 bis 10, **dadurch gekennzeichnet, dass** das Implantat mit einem Applikator lösbar verbunden ist, über den eine Einkopplung von HF-Energie möglich ist, die zu einer Koagulation des Implantates führt.

12. Implantat nach Anspruch 11, **dadurch gekennzeichnet, dass** der Applikator ein Biopsiegerät ist, das derart ausgebildet ist, dass gleichzeitig eine Gewebeprobe entnommen und das Implantat platziert werden kann.

13. Implantat nach einem der Ansprüche 2 bis 12, **dadurch gekennzeichnet, dass** die Antenne aus resorbierbaren Materialien besteht.

14. Implantat nach einem der Ansprüche 1 bis 6, **dadurch gekennzeichnet, dass** das zu fixierende Implantat eine Medikamentenpumpe oder ein Medikamentendepot ist.

15. Implantat nach einem der Ansprüche 1 bis 6, **dadurch gekennzeichnet, dass** das zu fixierende Implantat ein aktives oder aber ein passives Sensorimplantat zur Überwachung eines oder mehrerer physiologischer Parameter ist.

16. Implantat nach einem der Ansprüche 1 bis 6, **dadurch gekennzeichnet, dass** das zu fixierende Implantat ist ein Energiewandler für die Hyperthermietherapie ist, wobei entweder der gleiche Mechanismus für die Energiewandlung für die Implantatsfixierung und die Hyperthermie genutzt wird oder aber unterschiedliche Mechanismen für Fixation und Hyperthermie eingesetzt werden.
